Europäisches Patentamt

European Patent Office

Office européen des brevets

(19)

(11) **EP 1 206 267 B1**

(12) **EUROPÄISCHE PATENTSCHRIFT**

(45) Veröffentlichungstag und Bekanntmachung des Hinweises auf die Patenterteilung:
**29.09.2004  Patentblatt 2004/40**

(51) Int Cl.⁷: **A61K 31/70**, A61P 31/22, A61P 35/04

(21) Anmeldenummer: **00991044.9**

(22) Anmeldetag: **24.08.2000**

(86) Internationale Anmeldenummer:
**PCT/EP2000/008279**

(87) Internationale Veröffentlichungsnummer:
**WO 2001/015704 (08.03.2001 Gazette 2001/10)**

(54) **ARZNEIMITTEL ENTHALTEND XENOGENE OLIGO- ODER/UND POLYRIBONUKLEOTIDE**

MEDICAMENTS CONTAINING XENOGENIC OLIGO- OR/AND POLYRIBONUCLEOTIDES

MEDICAMENTS CONTENANT DES OLIGORIBONUCLEOTIDES ET/OU DES POLYRIBONUCLEOTIDES XENOGENES

(84) Benannte Vertragsstaaten:
**AT BE CH CY DE DK ES FI FR GB GR IE IT LI LU MC NL PT SE**
Benannte Erstreckungsstaaten:
**LT LV SI**

(30) Priorität: **27.08.1999  DE 19940748**

(43) Veröffentlichungstag der Anmeldung:
**22.05.2002  Patentblatt 2002/21**

(73) Patentinhaber: **SEINFELD, Hugo**
**80539 München (DE)**

(72) Erfinder: **SEINFELD, Hugo**
**80539 München (DE)**

(74) Vertreter: **Huber, Bernhard, Dipl.-Chem. et al**
**Weickmann & Weickmann**
**Patentanwälte**
**Postfach 86 08 20**
**81635 München (DE)**

(56) Entgegenhaltungen:
**DE-A- 2 547 696          FR-A- 2 713 487**
**US-A- 4 213 970**

**Beschreibung**

Die Erfindung betrifft die Verwendung xenogener Oligo- oder/und Polyribonukleotide zur Herstellung von Arzneimitteln für die Behandlung von Infektionen durch Herpesviridae und Hautmalignitäten.

Hintergrund der Erfindung

Viren aus der Familie der Herpesviridae sind weltweit verbreitete Pathogene, für welche die meisten Vertebraten anfällig sind. Die wichtigsten humanen Herpesviren sind Herpes Simplex Virus 1 und 2 (HSV-1,-HSV-2), Varicella Zoster Virus (VZV) und Humaner Cytomegalovirus (HCMV). HSV ruft in immunokompetenten Individuen Läsionen der Haut oder Schleimhäute hervor, die als Rezidive mit unterschiedlicher Häufigkeitimmer wiederkehren können. Man unterscheidet verschiedene Herpesviren nach der Lokalität der Läsionen, z.B. Herpes labialis oder Herpes genitalis etc.

Die bisherigen Behandlungsmethoden für solche Viren zielen hauptsächlich auf eine Hemmung der viralen. Replikation ab, z.B. mit Acyclovir, als bekannter Inhibitor der viralen DNA-Poiymerase. Allerdings kann das Virus mit der Zeit resistent gegen Acyclovir werden, dies trifft insbesondere für Herpes Simplex zu. Zudem verschaffen herkömmliche Mittel zwar Linderung bei akuten Läsionen, können jedoch Rezidive nicht wirksam verhindern.

Ende der 60er und anfangs der 70er Jahre fand man im Rahmen der Transplantationsforschung, daß mit xenogenen heterogenen Nukleinsäuren vorbehandeltes Gewebe oder schwache Antigene in verschieaenen immunologischen Untersuchungsmethoden wesentlich erhöhte Antititer aufwiesen. Diese Ergebnisse wurden mit einer Anzahl verschiedener Antigene in *in vitro* und *in vivo* Untersuchungen weiter bestätigt. Es gab jedoch keine Hinweise, daß Nukleinsäuren und insbesondere Oligooder/und Polyribonukleotide xenogenen Ursprungs zur Bekämpfung von viralen Infektionen geeignet sein könnten.

Vor allem in den USA wurden zur gleichen Zeit Versuche mit definierten synthetischen Poly- und Oligonukleotiden, besonders Ribonukleotiden angestellt, die aber wegen der hohen Toxizität in vivo nicht weiter verfolgt wurden.

Aus der US 4,213,970 ist ein tRNA-Präparat, welches auch DNA enthält, als antivirales Mittel bekannt. Dieses Mittel wird vor allen in wässrigem Medium eingesetzt und muss täglich oder alle zwei Tage erneut angewendet werden. Die Möglichkeit einer Rezidivverhinderung lässt sich daraus nicht entnehmen. In der FR 2,713,487 wird ein homöopatisches Mittel zur Behandlung von u.a. Infektionskrankheiten beschrieben, welches in wässriger Lösung typischerweise eine Verdünnung von $10^{-36}$ aufweist, sodass die darin enthaltene Nukleinsäure, bei der es sich um DNA oder RNA handeln kann, theoretisch gar nicht mehr vorhanden sein kann.

Aufgabe der vorliegenden Erfindung war daher die Bereitstellung eines Arzneimittels, das zur Behandlung von Herpesviridae-Infektionen, sowie von malignen Hauterkrankungen geeignet ist. Weiterhin war es eine Aufgabe der Erfindung, ein Arzneimittel bereitzustellen, welches die Rezidivrate bei Läsionen der Haut senkt, insbesondere bei viral verursachten Läsionen.

Die Aufgabe wird erfindungsgemäß gelöst durch die Verwendung von xenogenen Oligo- oder/und Polyribonukleotiden zur Herstellung eines wasserfreien Arzneimittels zur topischen Behandlung von Infektionen durch Herpesviridae oder/und Hauttumoren, wobei das Arzneimittel pro Rezidive einmal angewendet wird.

Xenogen bedeutet im Sinne der vorliegenden Erfindung, daß die Ribonukleinsäure aus einem anderen als dem damit zu behandeinden Organismus stammt, also solche Oligo- oder/und Polyribonukleotide, welche nicht aus demselben Organismus stammen, dem das Arzneimittel verabreicht werden soll. Bevorzugt handelt es sich bei den erfindungsgemäß verwendeten xenogenen Oligo- oder/und Polyribonukleotiden um solche aus Tiergeweben (z.B. Rindergewebe, fötales Kälbergewebe), Pflanzen und Einzellern, vorzugsweise aus Hefezellen (insbesondere Saccharomyces cerevisiae). Es werden bevorzugt Oligo- oder/und Polyribonukleotide aus Organismen verwendet, die entwicklungsgeschichtlich dem zu behandeinden Organismus möglichst fernstehen. Bei Arzneimitteln für den Menschen wird somit vorzugsweise RNA aus Tiergeweben oder besonders bevorzugt aus Pflanzen oder Einzellern, wie etwa Hefe, verwendet.

Der Erfindung liegen Untersuchungen mit RNA-Präparationen bei Herpes-Infektionen zugrunde. Dabei stellte sich heraus, daß das Auftragen von isolierter xenogener RNA auf Hautläsionen von Patienten mit Herpes Simplex labialis, Herpes Simplex cruris disseminata und Herpes Simplex genitalis, abgesehen von der immediaten Wirkung auf die Läsionen selbst, darüberhinaus überraschenderweise auch die Rezidivrate bei Patienten, die während des Jahres unter häufig wiederkehrenden Rezidiven litten, signifikant senkte. Dann wurde gefunden, daß die genannte RNA auch bei Hauttumoren z.B. Basaliomen ähnlich wirksam ist.

Die erfindungsgemäß verwendeten Oligo- oder/und Polyribonukleotide sind untoxisch und allein nicht antigen.

Es können Präparationen der Gesamt-RNA und deren Salze und Verbindungen wirksam eingesetzt werden. Besonders ist tRNA bevorzugt. Als Art der Gewinnung von erfindungsgemäß verwendbaren RNAs ist insbesondere die Phenolextraktion bevorzugt, speziell die hierin als Methode I und II bezeichneten Verfahren.

Die wirksame Menge der xenogenen Oligo- oder/und Polyribonukleotide per Dosierung ist bei jedem Patienten von verschiedenen Faktoren abhängig, z.B Lokalisation der Läsionen oder Größe und Ausdehnung der betroffenen Fläche, sowie der Art der Verabreichung. Die Dosierbreite liegt ab 0,1 mg aufwärts pro Dosiseinheit. Die untere Mengengrenze pro Dosiseinheit liegt bevorzugt bei mindestens 0,5 mg, stärker bevorzugt bei mindestens 2 mg, noch stärker bevorzugt bei mindestens 5 mg; und die obere Grenze liegt bevorzugt bei 5 mg, stärker bevorzugt bei 20 mg, noch stärker bevorzugt bei 10 mg.

Das Arzneimittel der Erfindung enthält die xenogenen Oligooder/und Polyribonukleotide in wesentlich wasserfreier Form, beispielsweise als Flocken, Pulver, Granulat, Salbe oder dgl.

Zusätzlich kann das erfindungsgemäße Arzneimittel physiologisch annehmbare Träger-, Hilfs-, Verdünnungsoder/und Zusatzstoffe oder/und Adjuvantien umfassen.

Die galenischen Zusammensetzungen, die die erfindungsgemäßen xenogenen Oligo- oder/und Polyribonukleotide enthalten, können für eine orale Anwendung als Tabletten, Lutsch- und Kautabletten, flüssige Suspensionen, in Pulverform oder Granulaten, Emulsionen, in harten oder weichen Kapseln, in Syrup oder Elixier, als Retardform oder als osmotische Kapsel für eine langsame Freigabe konfektioniert sein.

Eine andere galenische Form mit besonders vorteilhafter Wirkung sind wasserfreie Salben aus PEG-Mischungen.

Die Verabreichung erfolgt vorzugsweise topisch, aber auch oral, parenteral, rektal oder durch Inhalation. Der Ausdruck parenteral bezieht sich hier auf subkutane, intravenöse, intramuskuläre und intrasternale Injektionen oder Infusionstechniken.

Für die topische Anwendung wird die angewendete Gesamt-RNA odertRNA bevorzugt als Pulver oder PEG-Salbe (also in wasserfreier Form) auf die betroffene Stelle aufgebracht, bei Pulver gegebenenfalls kann die Haut leicht angefeuchtet werden. Diese läßt man bevorzugt an der Luft offen austrocknen.

Eine bevorzugte Ausführungsform der Erfindung ist eine Arzneimittelformulierung zur Behandlung von durch Herpesviridae verursachten Krankheiten, das auch die Häufigkeit von Rezidiven bei diesen Krankheiten vermindert. Besonders bevorzugt ist das erfindungsgemäß hergestellte Mittel zur Behandlung von durch Herpes Simplex Viren und Herpes Zoster (VZV) hervorgerufenen Läsionen, z.B. bei Läsionen und Rezidiven, die von Herpes Simplex labialis (Lippenbläschen) und genitalis verursacht werden und zur Behandlung von Hautmalignitäten, wie etwa Basaliomen, geeignet.

Vorzugsweise wird jeweils bei einer Lasten oder einer Rezidive eine Behandlung so früh wie möglich durchgeführt, wobei eine einmalige Anwendung bereits die Häufigkeit des Wiederauftretens vermindert.

Zusätzlich zur Therapierung von Menschen mit den xenogenen Oligooder/und Polyribonukleotiden,g.emäß.. der Erfindung können auch Warmblütler wie z.B. Pferde, Rinder, Schafe etc. so behandelt werden.

Die Erfindung wird weiter durch die nachfolgenden Beispiele und Versuchsergebnisse erläutert.

Beispiele

Beispiel 1

Gewinnung der erfindungsgemäß verwendbaren Oligo- oder/und Polyribonukleotide

Die einschlägige Literatur beschreibt zahlreiche Methoden zur Gewinnung von Nucleinsäuren, Nukleotiden und Nukleosiden, die jedem einschlägig Erfahrenen bekannt sind. Zwei Methoden mit geringen Modifikationen, beide auf Phenolisierung beruhend, kommen hier vorzugsweise zur Anwendung, Methode I zur Gewinnung der Gesamt-RNA (Georgiev, G. P. und Mantieva, V. L., Biochim. Biophys. Acta 61, 153 (1962)) und Methode II zur Gewinnung der tRNA (Bauer, S. et al., Biotechnology and Bioengineering 15, 1081 (1973)). Beide Methoden dienen der Extraktion größerer Mengen.

Methode I

Eine 15 % Suspension von Bierhefe (Saccharomyces cerevisiae) wurde in Puffer (A) [0,001 M EDTA, 0,01 M Tris-HCl Puffer, pH 5-6, 25 % Sucrose, 0,5% SDS (Natriumdodecylsulfat), 0,3% Na-Desoxycholat] wurde in einem Waring Blendor bei 10 °C mit 3000 UPM 3 Minuten lang homogenisiert. Das Homogenat wurde mit dem gleichen Volumen der Lösung (B) [ 80% rekristallisiertes Phenol in Puffer (A), 0,1 % 8-Hydroxychinolin, 1,2 % Diethylpyrocarbonat] versetzt und dann 30 Minuten bei 60 °C langsam gerührt. Alle Pufferlösungen wurden mit deionisiertem Wasser hergestellt, das vorher mit Bentonit aufgeschüttelt worden war. Danach wurde das phenolisierte Homogenat bei Raumtemperatur, ca. 20 °C, 15 Minuten mit 10.000 g zentrifugiert. Die wäßrige Phase wurde abgezogen, die Phenolund die Zwischenphase wurden verworfen. Die wäßrige Phase wurde mit dem gleichen Volumen eines 1:1 1 Gemisches der Lösung (B) und Chloroform-Isoamylalkohol (96:4) versetzt und wie oben beschrieben extrahiert. Die wäßrige Phase wurde 3 Mal mit einem halben Volumen Diethylether ausgeschüttelt, um das retliche Phenol zu entfernen. Die Lösung wurde auf 2% Natriumacetat gebracht und die RNA mit 2,5 Volumina absoluten Ethanols ausgefällt.

Die gefällte RNA wurde bei O°C und 5000 UPM abzentrifugiert und in einem eiskalten 0,01 M Tris-HCl Puffer, pH 7,0, und 0,001 M MgCl$_2$ aufgenommen. Zum Abbau eventueller DNA wurde die Lösung mit elektrophoretisch reiner pankreatischer DNase (4 μg/ml) versetzt und während 3 Stunden bei 22°C inkubiert. Dann wurden Proteinreste, die DNase und die RNasen mit Pronase (10 μg/ml) während 3 Stunden bei 37°C verdaut. Während dieser Zeit wurde auch die Pronase durch Selbstverdauung zerstört. Die RNA-Lösung wurde wie oben beschrieben mit der Lösung (B) 20 Minuten bei 60°C unter sanftem Rühren extrahiert, die Phasen durch Zentrifugation getrennt, die wäßrige Phase abgezogen und mit Diethylether ausgeschüttelt. Nach Zusatz von Natriumacetat (Endkonzentration 2%) wurde die RNA mit 2,5 Volumina Ethanol gefällt und abzentrifugiert. Der Niederschlag wurde in kaltem 2 %-igen Natriumacetat aufgenommen, mit 2,5 Volumina Ethylalkohol gefällt und über Nacht bei -20°C im Alkoholgemisch stehen gelassen. Dann wurde das Präzipitat abzentrifugiert, zweimal mit 75 %-igem, zweimal mit absolutem Ethanol und zweimal mit Diethylether gewaschen. Nach Trocknen im Wärmeschrank wurde eine lockere, trockene RNA erhalten, die in einem.dunklen Glasgefäß bei Raumtemperatur gelagert wurde.

Methode II

Diese Methode dient auch zur Extraktion großer Hefemengen (Kilogrammengen).

Ein gegebenes Gewicht Hefe wurde in der vierfachen Menge Puffer (A) (s. oben Methode I) im Kälteraum homogenisiert. Dem Homogenat wurden 40% Vol./Vol. der Phenollösung (B) und 5 % Gew./Vol. Eiswürfel aus deionisiertem Wasser zugesetzt und 30 Minuten lang gerührt. Der Überstand wurde abgesaugt und noch zweimal, wie unter Methode I beschrieben, phenolisiert. Die wäßrigen Überstände wurden in einem Gefäß gesammelt, in dem sich eine DEAE-Cellulose-Aufschwemmung (ca. 10 % Gew./Vol., Whatman DE-22), entsprechend dem halben Volumen der gesammelten Überstände, befand. Die DEAE Aufschwemmung wurde 30 Minuten durch Rühren in Suspension gehalten. Dann ließ man die DEAE während einer Stunde sedimentieren. Der Überstand wurde abgesaugt. Inzwischen wurden die Zwischen- und die Phenolphase noch zweimal mit der aliquoten Menge der Lösung (C) (83 % deionisiertes Wasser, 15 % Gew./Vol. Eiswürfel, 2 % Mg-acetat-Konzentrat [0,5M Mg-acetat in 0,25 Mercaptoethanol] 30 Minuten lang gerührt und dann 70-80 Minuten separieren gelassen. Die wäßrigen Überstände wurden in das Gefäß mit der DEAE übertragen, wieder gerührt und sedimentieren gelassen. Der Überstand wurde abgesaugt und die DEAE, wie oben, erst mit Lösung C zweimal, dann nochmals mit Lösung (D) (2 Volumen Mg-azetat Konzentrat, 2 Volumen NaCl-Konzentrat [3,75 M NaCl in Wasser], 0,2 Volumen Tris-HCl Konzentrat [2,5 M Tris-HCl, pH 7,5 in Wasser, 96 Volumen Wasser]) gewaschen.

Die DEAE-Cellulose wurde dann in eine Säule gepackt, die unten abgeschlossen war. Alle weiteren Schritte wurden im Kaltraum bei 4°C ausgeführt. Die Säule wurde mit der 12-fachen Menge des Säuleninhalts der Lösung (D), Flußgeschwindigkeit 1, 4 l /h, (nur durch Schwerkraft) gewaschen. Dann wurde die tRNA mit Lösung E [2 Volumen Mg-acetat-Konzentrat, 0,2 Volumen Tris-HCl-Konzentrat, 14 Volumen Na-Cl-Konzentrat, und 84 Volumen Wasser, Endkonzentration von NaCl 0,525 M, mit einem Fluß von 3 l/h eluiert. Die Fraktionen, die mehr als 35 A$_{260\ nm}$ Einheiten/ ml enthielten, wurden vereint und mit 1,5 Volumen Ethanol ausgefällt. Der weitere Vorgang entsprach Methode I.

Alternativ kann das letzte Präzipitat in Wasser aufgenommen und lyophilisiert werden.

Eine Variante dieser Methode ist die übliche Phenolisierung des Ausgangsmaterials: Aus der Oberphase wird die Roh-tRNA mit Isopropanol gefällt. Der Niederschlag wird nach der Zentrifugation mit dem Natriumacetatpuffer extrahiert und an der DEAE-Zellulose chromatographiert. Die Elution erfolgt mit dem Natriumacetat/Natriumchlorid-Gradienten, wie sie den in der Materie bewanderten Biochemikern bekannt ist. Die geeigneten Fraktionen, s. oben, werden mittels Quotientenmessung bestimmt und vereinigt. Die tRNA wird mit Ethanol gefällt, der Niederschlag wie oben aufgenommen und vorzugsweise lyophilisiert.

Die folgenden Tests wurden zur Untersuchung der Reinheit der Gesamt-RNA und der tRNA und zu deren Charakterisierungen angewendet:

Eiweiß wurde nach Lowry, O.H. et al. (J. Biol. Chem. 193, 265 (1951)) und durch A$_{260}$/A$_{280} \cong 2$, DNA nach Dische (Mikrochemie 8, 4 (1930), Gesamt-RNA nach Mejbaum (Physiol. Chem. 258, 117 (1939)), quantitative Bestimmung der tRNA und des Aminosäureneinbaus nach Sprinzl und Sternbach (Methods in Enzymology 59, 182 (1979)), Toxizität nach M. Nöldner (persönliche Mitteilung), Pyrogenfreiheit *in vitro* nach DAB 1997 (LAL-Test) und *in vivo* nach Ph.Eur. / DAB 1997 bestimmt.

Ergebnisse der Untersuchungen:

(Eigenschaften der Gesamt-RNA und der tRNA, Durchschnittswerte aus zehn Testungen)

Absorption

$$A_{260}/A_{280} \cong 1{,}94 - 2{,}0$$

| C, H, N Analyse | | |
|---|---|---|
| C | 32,67 | 32,42 |
| H | 5,22 | 5,20 |
| N | 2,29 | 2,00 |

mit korrespondierenden Werten verschiedener Gesamt- und tRNAs.

UV und IR Spektren

Die UV- und IR-Spektren variieren, sie sind fast gleich, aber nicht identisch, entsprechend biologischen Substanzen.

Molekulargewicht

Gesamt- und tRNA aus Hefe $\cong$ 22000-27000 Dalton Durchschnittswert, variierend bei veschiedenen Präparationen ;

| Protein | DNA (Gesamtinhalt) |
|---|---|
| 2,3% | neg. Gesamt-RNA Saccharomyces cerevisiae |
| 1,9% | neg. tRNA Saccharomyces cerevisiae |
| 0,9% | neg. Gesamt-RNA bovinen Ursprungs |

Durchschnittliche, allgemein übliche Qualität. Verbessete Reinheit brachte bei unverhältnismäßigem höheren Aufwand keine signifikant verbesserte therapeutische Wirkung.

Aminosäureneinbau bei tRNA, Mittelwert von 10 Untersuchungen

Lysin    69 - 85         pMol / $A_{260}$ Einheit
Phe      41 - 55
Ser      39 - 50
Val      77 - 90

Diese Mittelwerte ändern sich bei Hefen verschiedener Lots im angegebenen Rahmen.

Toxizität

Prüfung auf akute Toxizität an der Maus:

| Tiere | NMRI Mäuse, männlich, Fa. Janvier, Frankreich |
|---|---|

(fortgesetzt)

| Applikation | intravenös in eine Schwanzvene |
| --- | --- |
| Beobachtungsdauer | 24 Stunden |
| Stichprobenumfang | n = 10 in der höchsten Konzentration |
| Testsubstanz | a. bovine Gesamt RNA |
| | b. tRNA aus Biehefe (Saccharomyces cerevisiae) |
| Lösungsmittel | 0,9% NaCl in Wasser p.i. |

Ergebnis:

Bis zu einer maximalen Dosierung von 1g/kg/10ml i.v. zeigten die Versuchstiere innerhalb des Beobachtungszeitraums von 24 Stunden keinerlei Auffälligkeiten.

Pyrogenfreiheit

A. Der Pyrogengehalt der Gesamt-RNA sowie der t-RNA, beide wie bisher beschrieben, wurde mit dem In-vitro-Test auf Endotoxine nach DAB 1997 (LAL TEST) und an Kaninchen nach Ph. Eur. /DAB 1997 bestimmt.

1. Gesamt-RNA
Endotoxin Standard EC 5
Amoebozytenlysat

- Deklarierte Empfindlichkeit:     0,06 EU/ml
- Gefundene Empfindlichkeit:     0,06 EU/ml

Prüflösung: 100mg RNA gelöst in 20ml Wasser-LAL (0,5%)
Ergebnis:
Der Endotoxingehalt der Prüflösung 0,5% 1:5 mit Wasser-LAL verdünnt: < 0,03 EU/ml.
2. tRNA
Endotoxin Standard EC 5
Amoebozytenlysat

- Deklarierte Empfindlichkeit:     0,06 EU/ml
- Gefundene Empfindlichkeit:     0,06 EU/ml

Prüflösung: 100mg RNA gelöst in 20ml Wasser-LAL (0,5%)
Ergebnis:
Der Endotoxingehalt der Prüflösung 0,5% 1:10 mit Wasser-LAL verdünnt: < 0,03 EU/ml.

B. In Vivo Prüfung auf Pyrogenfreiheit nach Ph.Eur. /DAB 1997

1. Gesamt-RNA

| Prüflösung | 1% der Testsubstanz in pyrogenfreiem Wasser p.i. |
| --- | --- |
| Dosis | 1,0 ml/Tier |
| Tiere | 3 Kaninchen, entsprechend DAB 1997 |

Ergebnis:
Summe der Temperaturdifferenzen von 3 Kaninchen war 1,05 °C, Pyrogene sind somit nicht nachweisbar.

2. tRNA

| Prüflösung | 1% der Testsubstanz in pyrogenfreiem Wasser p.i. |
| --- | --- |

(fortgesetzt)

| Dosis | 1,0 ml/Tier |
|-------|-------------|
| Tiere | 2 mal 6 Kaninchen, entsprechend DAB 1997 |

Ergebnis:

a. Summe der Temperaturdifferenzen von 6 Kaninchen: 5,40 °C

b. Summe der Temperaturdifferenzen von 6 Kaninchen: 4,10 °C, Pyrogene nachweisbar.

Beispiel 2

Nachweise der Wirksamkeit der Substanzen dieser Erfindung

70 Patienten, davon 40 mit Herpes Simplex I (H. labialis und 30 Patienten mit Herpes Simplex II (H. genitalis), alle mit häufigen Rezidiven wurden mit Gesamt-RNA behandelt. Die RNA entstammte aus Extraktionen von bovinem fötalem Gewebe, ausgenommen Leber. Die pulverförmige RNA wurde auf die leicht angefeuchteten Läsionen, 5 bis 10 mg je nach Größe der Läsion, aufgebracht und trocknen gelassen. Alle Patienten wurden über 1 Jahr beobachtet.

5 Patienten waren in Bezug auf Rezidiven Non-Responder, 7 Patienten konnten wegen mangelnder Compliance nicht ausgewertet werden. Der Rest der Patienten, die sonst jährlich mehrere Rezidiven hatten, wiesen einen signifikanten Rückgang der Rezidiven auf. Die Auswertung erfolgte mittels des non-parametrischen Mann-Whitney U Tests. Die Signifikanz der Ergebnisse war p < 0,001. (SPSS, Npar, Mann-Whitney U-Test)

In einer Doppelblindstudie mit einer Beobachtungszeit von 1 Jahr wurden zwei Gruppen von je 100 Patienten mit Herpes Simplex labialis und Herpes Simplex genitalis mit mehr als 4 Rezidiven pro Jahr mit boviner Gesamt-RNA wie oben oder mit tRNA aus Bierhefe behandelt. Die Auswertung erfolgte nach einem Jahr mit dem Programm SPSS, Npar TEST: Mann-Whitney und $X^2$-Test.

Im Vergleich mit den Placebopatienten war war der Rückgang der Rezidiven hochsignifikant: bei beiden war p < 0,001. Der Unterschied zwischen den beiden RNA war nicht groß.

Diese Ergebnisse rechtfertigen den Einsatz der RNA bei Patienten, besonders da keinerlei Nebenwirkungen oder toxische Erscheinungen über mehrere Jahre zu beobachten waren.

Bei Anwendung der beschriebenen Substanzen bei facialem Herpes Simplex bei Patienten, die auch ein faciales Basaliom hatten, wurde festgestellt, daß dieses zurückging. Daher umfaßt die Indikation des erfindungsgemäßen Mittels auch Malignitäten.

**Patentansprüche**

1.   Verwendung von xenogenen Oligo- oder/und Polyribonukleotiden zur Herstellung eines wasserfreien Arzneimittels zur topischen Behandlung von Infektionen durch Herpesviridae oder/und Hauttumoren, wobei das Arzneimittel pro Rezidive einmal angewendet wird.

2.   Verwendung nach Anspruch 1,
**dadurch gekennzeichnet,**
**dass** das Arzneimittel zusätzlich physiologisch annehmbare Träger-, Hilfs-, Verdünnungs- oder/und Zusatzstoffe umfasst.

3.   Verwendung nach Anspruch 1 oder 2,
**dadurch gekennzeichnet,**
**dass** die xenogenen Oligo- oder/und Polyribonukleotide aus Organismen stammen, die entwicklungsgeschichtlich dem zu behandelnden Organismus fern stehen.

4.   Verwendung gemäß einem der vorhergehenden Ansprüche zur Behandlung von durch Herpes Simplex Virus oder/und Varicella Zoster Virus hervorgerufenen Läsionen der Haut oder/und Schleimhaut.

5.   Verwendung von xenogenen Oligo- oder/und Polyribonukleotiden zur Herstellung eines wasserfreien Arzneimittels für die Behandlung von Infektionen durch Herpesviridae oder/und Hauttumoren durch Verabreichung in einer wirksamen Menge von 0,1 mg aufwärts pro Dosiseinheit einmal pro Rezidive.

**Claims**

1. Use of xenogeneic oligo- and/or polyribonucleotides for producing an anhydrous medicament for the topical treatment of infections by Herpesviridae and/or skin tumours, the medicament being applied once per recurrence.

2. Use according to Claim 1,
**characterized in that**
the medicament additionally comprises physiologically acceptable carriers, excipients, diluents and/or additives.

3. Use according to Claim 1 or 2,
**characterized in that**
the xenogeneic oligo- and/or polyribonucleotides originate from organisms which are evolutionarily distant from the organism to be treated.

4. Use according to any of the preceding claims for the treatment of lesions of the skin and/or mucosa, caused by herpes simplex virus and/or varicella zoster virus.

5. Use of xenogeneic oligo- and/or polyribonucleotides for producing an anhydrous medicament for the treatment of infections by Herpesviridae and/or skin tumours by administering an active amount of 0.1 mg and higher per dose unit once per recurrence.

**Revendications**

1. Utilisation d'oligoribonucléotides et/ou polyribonucléotides xénogènes pour la fabrication d'un médicament anhydre pour le traitement topique d'infections herpétiques et/ou de tumeurs cutanées, où le médicament est utilisé en application unique à chaque récidive.

2. Utilisation selon la revendication 1, **caractérisée en ce que** le médicament contient également des excipients, des adjuvants, des diluants et/ou des additifs physiologiquement acceptables.

3. Utilisation selon la revendication 1 ou 2, **caractérisée en ce que** les oligoribonucléotides et/ou polyribonucléotides xénogènes proviennent d'organismes phylogénétiquement éloignés de l'organisme à traiter.

4. Utilisation selon l'une des revendications précédentes dans le traitement des lésions de la peau et/ou des muqueuses dues au virus herpès simplex (HSV) ou au virus zona-varicelle (VZV).

5. Utilisation des oligoribonucléotides et/ou polyribonucléotides pour la production d'un médicament anhydre dans le traitement des infections herpétiques et /ou des tumeurs cutanées via l'administration en quantité efficace de 0,1 mg en prise unique à chaque récidive.